# EUROPÄISCHE PATENTSCHRIFT

(11) **EP 2 143 456 B1**
(45) Veröffentlichungstag und Bekanntmachung des Hinweises auf die Patenterteilung: **03.08.2011**
(21) Anmeldenummer: 08159931.8
(22) Anmeldetag: 08.07.2008
(51) Int. Cl.: A61M 5/32, A61M 5/50

(54) **Kanülenschutz und Einwegspritzensystem**
Cannula protection and disposable injection system
Protection de canules et système de seringues à usage unique

(43) Veröffentlichungstag der Anmeldung: 13.01.2010
(73) Patentinhaber: Kunststofftechnik Waidhofen an der Thaya GmbH, 3830 Waidhofen a.d. Thaya (AT)
(72) Erfinder: Weist, Mario, 3830 Waidhofen (AT)
(74) Vertreter: Engel, Christoph Klaus

(56) Entgegenhaltungen:
- FR-A- 2 613 628
- US-A- 4 737 144
- US-A- 4 738 663
- US-A- 4 927 416
- US-A- 5 219 338
- US-A- 5 795 336
- US-A1- 2004 127 857

## Beschreibung

Die Erfindung betrifft einen Kanülenschutz gemäß dem Oberbegriff des Anspruchs 1. Dieser Kanülenschutz dient zur Abdeckung einer an einer Spritze angebrachten Kanüle, insbesondere um ungewollte Verletzungen an der Kanüle sowie eine Kontamination der Kanüle kurz vor deren Verwendung am Patienten zu vermeiden. Die Erfindung betrifft außerdem ein Einwegspritzensystem mit einem solche Kanülenschutz.

Der Kanülenschutz umfasst eine rohrförmige Hülse und einen Faltenbalg, welcher teilweise gemeinsam mit der Hülse zumindest den Bereich der Kanüle vollständig umschließt. Der Kanülenschutz umfasst auch eine radial wirkende Klemmfeder, welche dazu dient, die eingelegte Einwegspritze in der gewünschten Position zu halten, bzw. den Schutzmechanismus zu aktivieren.

Diverse Nadelschutzvorrichtungen sind aus dem Stand der Technik bekannt. Die kurzfristige Aufbewahrung von zur Injektion vorbereiteten Einwegspritzen bis zur Durchführung der Injektion, sowie die Entsorgung bereits benutzter Einwegspritzen ist aufgrund der Gefahr der Ansteckung mit verschiedenen Erregern und aufgrund der Verletzungsgefahr an der freiliegenden Kanüle von besonderer Bedeutung.

Aus der DE 100 44 383 C2 ist eine Nadelschutzvorrichtung bekannt, welche an einer auf eine Einwegspritze aufsteckbaren Kanüle verwendet wird. Die Nadelschutzvorrichtung umfasst einen Träger und eine Nadelhülle. Die Nadelhülle kann aus einer zurückgeschobenen Stellung in eine Schutzstellung, in der sie die Einstechnadel bis wenigsten zu der Nadelspitze umgibt, verbracht werden. Die Nadelhülle umfasst einen Falten balg und Blockierelemente, welche ein Blockierteleskop bilden, welches in einem ausgefahrenen Zustand eine Blockierstellung einnimmt. Diese Nadelschutzvorrichtung betrifft ausschließlich die Kanüle, welche nach Gebrauch einer Einwegspritze wieder von dieser entfernt werden kann.

Aus der EP 0 763 369 B1 ist eine Nadelschutzvorrichtung mit einer kollabierenden Hülle bekannt. Der Nadelschutz betrifft einen Katheter, bei welchem als kollabierende Hülle ein sich gegen eine Federkraft aufschiebbarer Faltenbalg verwendet wird. Die Hülle schirmt sicher und automatisch die scharfe Spitze der Einführungsnadel des Katheters ab, nachdem die Nadel zum Legen des Katheters bei einem Patienten benutzt worden ist. Die Schutzvorrichtung hat einen komplizierten Aufbau mit vielen Einzelteilen und einen komplizierten Feder-Rast-Mechanismus zur Aktivierung des Schutzmechanismus. Der Faltenbalg ist aus einem flexiblen undurchlässigen, nicht elastischen Material mit niedrigen Dehnungseigenschaften gebildet. Die Einzelteile der Schutzhülse werden aufwendig, beispielsweise durch Presspassung, Ultraschallschweißen oder Anwendung eines Standardklebers zusammengefügt.

Aus der US 2004/0127857 ist ein mehrteiliger Kanülenschutz mit einer äußeren Hülse und einer inneren Schutzhülse bekannt. Zwischen innerer und äußerer Hülse sind aufwendige mechanische Mittel vorgesehen, um die Hülsen zum Zweck der Injektion gegeneinander zu fixieren und zur manuellen oder automatischen Freigabe eines Rückstellmechanismus, um die Nadel innerhalb der äußeren Hülse zu "versenken". In einer Ausführungsform ist in der inneren Hülse eine Spiralfeder integral mit der Hülse gefertigt.

Die Aufgabe wird durch einer Kanülenschutz gemäß dem beigefügten Anspruch 1 und durch ein Einwegspritzensystem gemäß Anspruch 10 gelöst.

Ein erfindungsgemäßer Kanülenschutz weist eine rohrförmige Hülse zur axialbeweglichen Aufnahme einer Spritze, vorzugsweise einer Einwegspritze auf. Die rohrförmige Hülse ist an ihrem einen Ende durch eine Grundfläche verschlossen, wobei die Grundfläche eine Öffnung für den Durchlass einer auf die Einwegspritze aufgesteckten Kanüle aufweist. Am anderen Ende der Hülse ist ein stauchbares Federelement als Faltenbalg ausgeführt und einstückig angeformt, wobei der Faltenbalg eine Vorspannung aufweist, so dass er nach einem Zusammendrücken in axialer Richtung nach Entlastung seine ursprüngliche Form wieder einnimmt.

Hülse und Faltenbalg sind in der Gesamtlänge so dimensioniert, dass die Einwegspritze mit aufgesteckter Kanüle von dem Kanülenschutz in ihrer gesamten axialen Erstreckung zumindest im Bereich der Kanüle vollständig umschlossen ist. Der Faltenbalg muss insgesamt etwa um die Länge der Kanüle zusammendrückbar sein, damit diese bei eingelegter Einwegspritze zum Zwecke der Injektion im Wesentlichen vollständig aus der Öffnung austreten kann. Vorteilhafterweise wird der Kanülenschutz in verschiedenen Größen und mit jeweils verschieden langen Faltenbälgen bereitgehalten.

Die Hülsenlänge der Hülse ist kürzer als die Länge des Spritzenzylinders der einzulegenden Spritze. Die Stauchungslänge ist mindestens gleich der Länge der Kanüle der einzulegenden Spritze. Die Summe aus Hülsenlänge und gestreckter Länge des Faltenbalgs ist mindestens gleich der Summe aus Länge des Spritzenzylinders und Länge der Kanüle

Bei einem axial gerichteten Druck auf den Faltenbalg wird die Einwegspritze in dem Kanülenschutz verschoben, wodurch die Kanüle durch die Öffnung in der Grundfläche hindurch freigegeben wird.

Der Kanülenschutz umfasst weiterhin mindestens eine radial wirkende Klemmfeder, welche im Bereich der Hülse angeordnet ist. Vorzugsweise sind mehrere Klemmfedern um den Umfang der Hülse gleichmäßig verteilt vorgesehen.

Die Klemmfeder bzw. die Klemmfedern können mittels Fingerdruck zweier die Hülse greifender Finger betätigt werden, um die Klemmfeder bzw. die Klemmfedern gegen die eingelegte Einwegspritze zu drücken und diese in der augenblicklichen Position zu halten. Dies erfolgt zu Injektionszwecken, wenn die Kanüle aus der Öffnung hinausragt.

Erfindungsgemäß ist der Faltenbalg unter Druck, also beim Verschieben der Spritze mit der Kanüle in Richtung Öffnung, zusammenschiebbar und er nimmt federelastisch seine Ursprungsform nach der Entlastung wieder ein, nämlich wenn die Klemmfedern freigegeben werden, insbesondere beim Ablegen der Spritze.

Die Vorteile der Erfindung sind insbesondere darin zu sehen, dass der Kanülenschutz besonders einfach und preisgünstig, z. B. durch ein Spritzgießverfahren, herstellbar ist. Der erfindungsgemäße Kanülenschutz lässt sich in Verbindung mit herkömmlichen Spritzen verwenden, ohne dass deren Bauform verändert werden muss.

In einer besonders bevorzugten Ausführungsform der Erfindung weist der Faltenbalg eine Schraubenform auf. Diese ist vorzugsweise im Spritzgießverfahren durch einen Drehkern herstellbar, welcher nach dem Gießen aus dem fertigen Kanülenschutz herausgedreht wird. Auf diese Weise lässt sich der Kanülenschutz leicht entformen, ohne dass er in mehrere Teile zertrennt werden muss. Der gesamte Kanülenschutz kann daher einteilig ausgeformt sein.

Vorteilhafterweise sind an der Hülse zwei Klemmfedern radial gegenüberliegend, oder vier Klemmfedern gleichmäßig um den Umfang verteilt angeordnet. Es kann aber ebenso eine andere Anzahl an Klemmfedern gewählt werden. Die Klemmfedern sind einstückig mit der Hülse ausgebildet, indem zungenförmige Klemmfedern geformt werden, die zur leichteren Betätigung einen über die Wandung der Hülse hervorstehenden Abschnitt aufweisen. Die jeweils radial gegenüberliegenden Positionen zweier Klemmfedern begünstigen das Betätigen des Klemmmechanismus beim Greifen der Spritze mit zwei Fingern des Bedieners.

Vorzugsweise kann eine Skala der eingelegten Einwegspritze trotz umschließenden Kanülenschutzes beobachtet werden. Dies kann vorteilhafterweise durch einen oder mehrere axial verlaufende Schlitze in der Hülse erreicht werden. Es ist aber auch möglich, den Kanülenschutz vollständig oder abschnittsweise aus einem durchsichtigen Kunststoffmaterial zu fertigen.

Der Kanülenschutz ist erfindungsgemäß einstückig ausgebildet oder in axialer Richtung zweigeteilt und über einen Rastmechanismus zusammensteckbar.

Die Öffnung zum Durchlass der Kanüle hat vorzugsweise eine kreisrunde Form, aber auch andere Formen, wie beispielsweise die eines Kreuzes, sind möglich.

Ein besonders vorteilhaftes Einwegspritzensystem umfasst einen Kanülenschutz der zuvor beschriebenen Art und eine Einwegspritze, wobei die Einwegspritze oder ein Teil davon über eine Verbindungsraste mit dem Kanülenschutz unlösbar arretierbar ist. Das erfindungsgemäße Einwegspritzensystem verhindert eine weitere Verwendung der in den Kanülenschutz eingelegten Einwegspritze.

In einer bevorzugten Ausführungsform des Einwegspritzensystems ist der Kolben der Einwegspritze mit einer Sollbruchkante zur Vermeidung der Wiederverwendung versehen, und im Inneren des Spritzenzylinders ist nahe des Auslasses ein im Querschnitt verjüngter Steg vorgesehen, welcher die Sollbruchkante bei deren Auftreffen auf den Steg zerstört, sodass der Kolben nach Gebrauch nicht mehr dichtend in der Einwegspritze gelagert ist.

In einer zweiten bevorzugten Ausführungsform des Einwegspritzensystems ist an einem Betätigungselement der Einwegspritze und an dem Kanülenschutz ein Verschlussmechanismus vorgesehen, der nach einmaliger schließender Betätigung des Spritzenkolbens einrastet und dadurch eine wiederholte Benutzung der Einwegspritze verhindert.

Bevorzugte Ausführungsformen der Erfindung werden nachfolgend anhand der Figuren näher erläutert. Es zeigen:
- Fig. 1:: eine räumliche Ansicht eines Kanülenschutzes;
- Fig. 2:: eine Längsschnittdarstellung des Kanülenschutzes mit eingelegter Einwegspritze;
- Fig. 3:: eine räumliche Ansicht einer zweiten Ausführungsform des Kanülenschutzes;
- Fig. 4:: drei räumliche Detaildarstellungen eines zweiteilig ausgeführten Kanülenschutzes;
- Fig. 5:: eine Detailansicht eines Verschlussmechanismus eines Einwegspritzensystems;
- Fig. 6:: eine Detailansicht einer Einwegspritze.

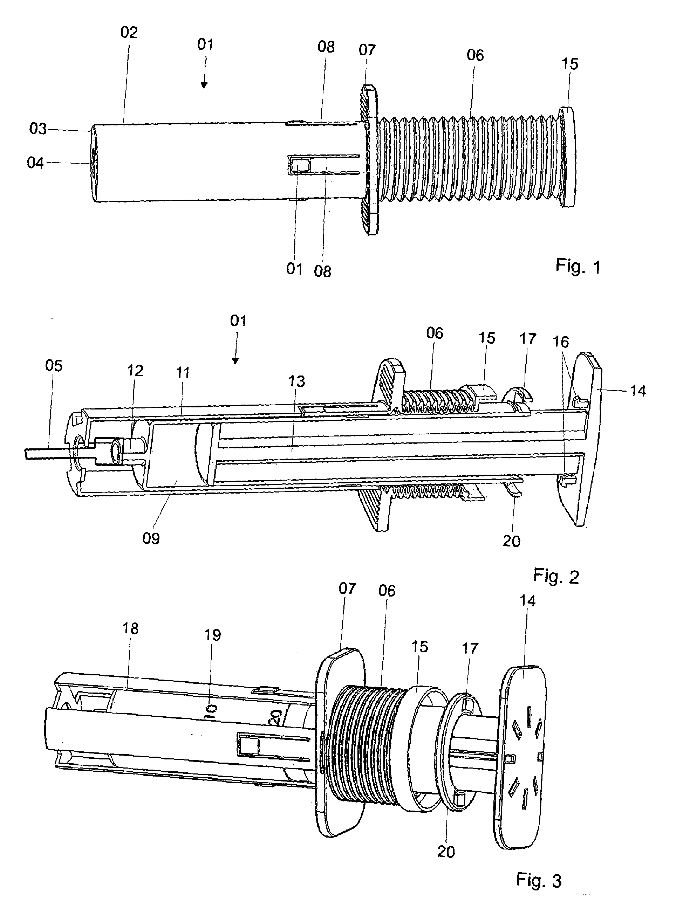

Fig. 1 zeigt einen Kanülenschutz 01 in einer räumlichen Darstellung. Der Kanülenschutz 01 umfasst eine rohrförmige Hülse 02. Die Hülse 02 ist an ihrem ersten Ende durch eine Grundfläche 03 verschlossen, welche eine Öffnung 04 für den Durchlass einer Kanüle 05 (Fig. 2) einer Einwegspritze 09 (Fig. 2) aufweist. Die dargestellte Öffnung 04 hat eine kreisrunde Form. Es ist aber ebenso möglich, andere geeignete Formen, wie beispielsweise ein Kreuz oder ähnliches, zu verwenden.

An einem der Grundfläche 03 gegenüberliegenden Ende der Hülse 02 ist ein Faltenbalg 06 einstückig angeformt. Der Faltenbalg 06 weist eine Vorspannung auf und ist in axialer Richtung unter Druck zusammenschiebbar bzw. stauchbar. Bei Entlastung nimmt der Faltenbalg 06 seine ursprüngliche Form wieder ein. Der Faltenbalg 06 ist vorzugsweise mit einer gewendelten Wand gebildet, sodass er wie eine Schraubenfeder arbeitet. Der Faltenbalg 06 ist hier in völlig gestrecktem Zustand dargestellt. Die vom Faltenbalg im gestauchten Zustand aufzubringende Rückstellkraft muss so groß sein, dass die Hülse 02 axial auf der Spritze nach vorn geleitet und die Kanüle aufnimmt, sobald vom Benutzer keine Kraft auf das gesamte System eingeprägt wird. Gleichzeitig soll die bei der Injektion zu überwindenden Gegenkraft des Faltenbalgs so klein wie möglich sein, um die Injektion nicht zu erschweren.

Zwischen der Hülse 02 und dem Faltenbalg 06 sind vorzugsweise zwei Vorsprünge 07 angeordnet, die dem Halten des Kanülenschutzes 01 mit zwei Fingern dienen, wenn die Einwegspritze in den Kanülenschutz eingesetzt wird oder während der Injektion.

Auf dem Umfang der Hülse 02 sind in der dargestellten Ausführungsform vier radial wirkende Klemmfedern 08 angeordnet, welche bei einem radial auf sie gerichteten Druck die eingelegte Einwegspritze in der augenblicklichen Position in der Hülse 02 halten, vorzugsweise mit zur Injektion freigelegter Kanüle.

Die Anordnung von vier Klemmfedern 08 um den Umfang verteilt hat sich als vorteilhaft herausgestellt, weil dadurch der Kanülenschutz 01 mit eingelegter Einwegspritze nicht gedreht werden muss, um mit zwei Fingern die Klemmfedern zu bedienen. Die gewohnte Einhandbedienung der Spritze ist auch mit dem Kanülenschutz 01 weiterhin möglich.

Die Klemmfedern 08 sind als federnde Zungen einstückig mit der Hülse 02 ausgebildet und besitzen für eine leichtere Betätigung einen radialen Überstand 10.

Fig. 2 zeigt eine Längsschnittdarstellung des erfindungsgemäßen Kanülenschutzes 01 mit einer darin eingelegten Einwegspritze 09. Die Einwegspritze 09 umfasst in bekannter Weise einen Spritzenzylinder 11 mit einer Düse 12 zum Aufstecken der Kanüle 05. Die Einwegspritze 09 umfasst weiterhin einen Spritzenkolben 13 mit einer Betätigungsplatte 14 zum Eindrücken des Spritzenkolbens 13 in den Spritzenzylinder 11.

Die gesamte Einwegspritze 09 ist gemeinsam mit der aufgesteckten Kanüle 05 innerhalb des Kanülenschutzes 01 axial verschiebbar und durch Drücken der Klemmelemente 08 in der jeweiligen Position festlegbar. Die Hülse 02 besitzt zwischen der Grundplatte 03 und dem Vorsprung 07 am anderen Ende eine Hülsenlänge, die kürzer als die Länge des Spritzenkobens 11 ist. Der Faltenbalg 06 ist in weitgehend zusammengeschobener Position dargestellt, die nur aufrecht erhalten werden kann, wenn der Nutzer die Klemmelemente 08 betätigt und damit die Spritze in der Hülse 02 festklemmt. Anders als in der in Fig. 2 zu Verständniszwecken gewählten Darstellung, schlägt das freie Ende 15 des Faltenbalgs 06 beim Betätigen der Spritze an einem Anschlagring 20 des Spritzenkolbens 11 an, sodass der Faltenbalg gestaucht wird, wenn auf den Spritzenkolben oder die Betätigungsplatte 14 des Spritzenkolbens eine Kraft in axialer Richtung ausgeübt wird. Die Reibung zwischen Spritzenzylinder 11 und Innenwand der Hülse 02 ist durch genügend Spiel so klein zu halten, dass die Spritze im Wesentlichen nur gegen den Widerstand des Faltenbalgs in die Hülse 02 geschoben werden kann.

Die Stauchungslänge des Faltenbalgs 06, um welcher diese mindestens zusammen gestaucht werden kann und um die er sich nach Wegfall der Stauchungskraft mindestens wieder ausdehnt, entspricht mindestens der Länge de Kanüle 05 (ggf. abzüglich ihres Sockels), um ein vollständiges Abdecken der Kanüle beim Ausdehnen des Faltenbalgs und der damit einhergehenden Verlagerung der Hülse 02 zu erreichen.

Vor der eigentlichen Injektion legt der Benutzer die Kanüle 05 frei, indem die Spritze in die Hülse 02 eingeschoben wird. Die Injektion kann dann wie gewohnt gesetzt werden. Legt der Nutzer die Spritze aus der Hand, entfällt die Klemmkraft, der Faltenbalg 06 entspannt sich und schiebt dabei die Hülse 02 über die Kanüle 05.

Der Spritzenkolben 13 ist in dem in Fig. 2 dargestellten Zustand weit in den Spritzenzylinder 11 eingeschoben. Dies entspricht dem Moment während der Injektion bzw. beim Aufziehen der Injektionslösung in die Spritze.

In der in Fig. 2 dargestellten Ausführungsform bildet der Kanülenschutz 01 gemeinsam mit der Einwegspritze 09 ein vorteilhaftes Einwegspritzensystem, welches bestückt mit der Kanüle 05 und gefüllt mit einer Injektionslösung auch in dieser komplettierten Form ausgeliefert werden kann.

Die Betätigungsplatte 14 des Spritzenkolbens 13 weist zum Zweck einer mechanischen Verriegelung nach Gebrauch zwei radial gegenüberliegend angeordnete Rasthaken 16 auf, welche nach vollständigem Einschieben des Spritzenkolben 13 in den Spritzenzylinder 11 in eine Rastnut 17 einrasten. In der dargestellten Ausführungsform ist die Rastnut 17 am Spritzenzylinder 11 der Einwegspritze 09 angeordnet, jedoch kann in einer besonders bevorzugten Ausführungsform die Rastnut 17 direkt am Ende des Faltenbalges 06 angeordnet werden, wodurch eine vorteilhafte nicht lösbare Verbindung zwischen Einwegspritze 09 und Kanülenschutz 01 nach Gebrauch der Einwegspritze 09 erreicht wird. Die Funktion des Kanülenschutzes ist durch das Einrasten der Rasthaken nicht beeinträchtigt, sodass nach erfolgter Injektion sowohl eine Verriegelung der Einwegspritze als auch eine Abdeckung der benutzten Kanüle gegeben ist.

Fig. 3 zeigt eine räumliche Darstellung des Einwegspritzensystems gemäß Fig. 2. Der Kanülenschutz 01 weist axial verlaufende Sichtschlitze 18, vorzugsweise vier auf dem Umfang verteilt, an, welche der Beobachtung einer Skala 19 der Einwegspritze 09 dienen. Die Beobachtungsmöglichkeit ist auch erforderlich, um beispielsweise das Einströmen von Blut in die Spritze beobachten zu können.

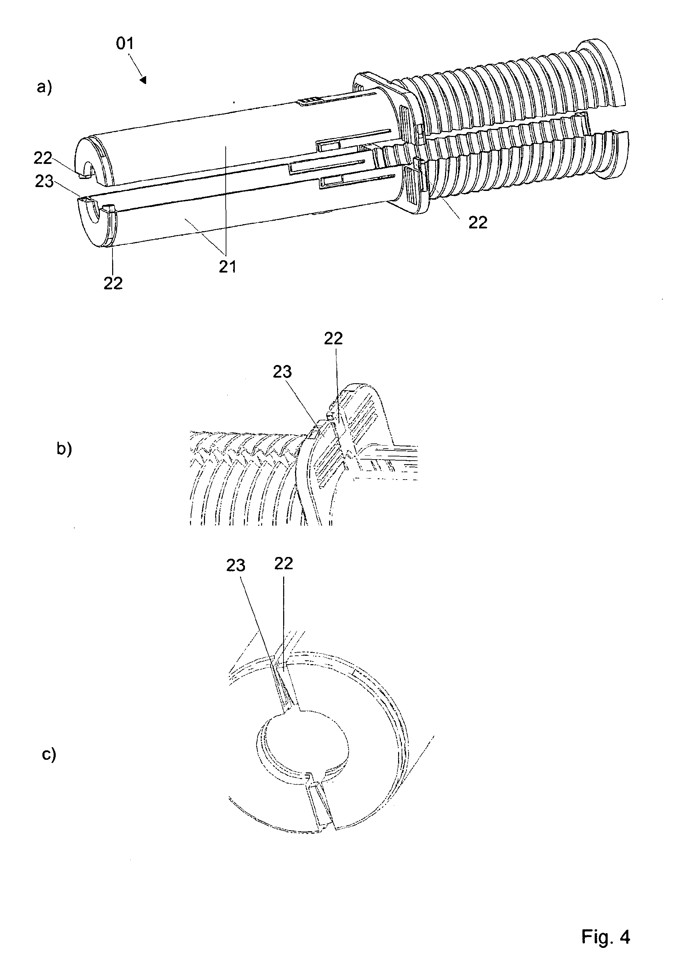

Fig. 4 zeigt in drei Ansichten einen in axialer Richtung zweigeteilten Kanülenschutz 01, der aus zwei Hälften 21 zusammengesteckt werden kann. Diese Ausführungsform ist besonders preisgünstig in der Herstellung, da die Spritzgussformen wesentlich einfacher ausgeführt werden können. Bei der zweiteiligen Gestaltung kann der Faltenbalg 06 in nicht gewendelter Struktur geformt werden.

In den Abb. b) und c) ist im Detail der Rastmechanismus zur Verbindung der Hälften 21 dargestellt. An vorzugsweise drei axial verteilten Verbindungsstellen weisen die beiden Hälften 21 gegenüberliegend jeweils eine Nase 22 und eine Nut 23 auf.

Eine fertig vorbereitete bzw. aufgezogene Einwegspritze kann vorteilhafterweise in eine der Hälften eingelegt werden und durch Zuschnappen der zweiten Hälfte verkapselt werden. Dadurch wird die Handhabung vereinfacht. Besondere Anforderungen an die Dichtigkeit werden an den Kanülenschutz 01 nicht gestellt, weswegen die zweiteilige Ausführungsform bevorzugt verwendet werden kann.

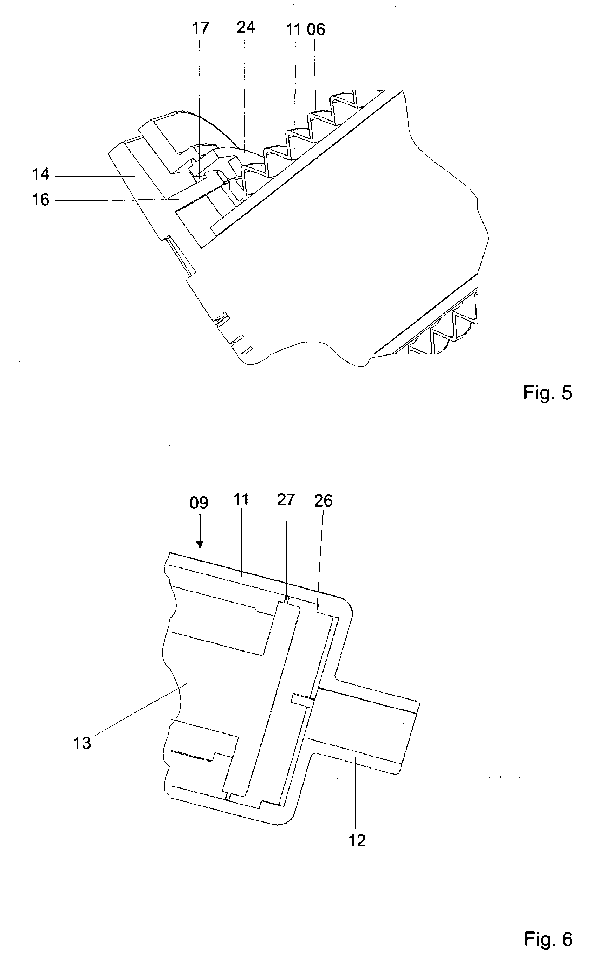

Fig. 5 zeigt eine Detailansicht eines Rastmechanismus eines Einwegspritzensystems ähnlich dem, wie es in Fig. 2 bereits beschrieben wurde. Zusätzlich zu der bereits beschriebenen Funktion weist der Faltenbalg 06 einen Fixierabschnitt 24 auf, welcher in die an dem Spritzenzylinder 11 der Einwegspritze 09 vorgesehenen Rastnut 17 bei der Montage einrastet. Der Rasthaken 16 des Spritzenkolbens 13 rastet nach vollständiger Betätigung ebenfalls in dieser Rastnut 17 ein, wodurch ein wiederholtes Öffnen oder Benutzen der Einwegspritze sicher verhindert wird.

Fig. 6 zeigt eine Detailansicht einer bevorzugten Ausführungsform einer in dem Einwegspritzensystem zu verwendenden Einwegspritze 09. Der Spritzenzylinder 11 weist in seinem Inneren nahe des mit der Düse 12 versehenen Auslasses einen Steg 26 auf, welcher mit einer am Spritzenkolben 13 vorgesehenen, im Querschnitt verjüngten Sollbruchkante 27 beim schließenden Betätigen der Einwegspritze 09 zusammentrifft und diese dabei zerstört. Durch das Zerstören der Sollbruchkante 27 ist der Spritzenkolben 13 nicht mehr dichtend im Spritzenzylinder 11 gelagert, wodurch ein erneutes Aufziehen einer Injektionslösung in die Einwegspritze nicht mehr möglich ist.

Die Kombination der beschriebenen Merkmale führt zu einem Einwegspritzensystem, welches hohen Hygieneanforderungen genügt. Die Verletzungsgefahr an der Kanüle ist minimiert, da die Kanüle durch den Kanülenschutz gesichert ist, sobald die Spritze aus der Hand gelegt wird. Gleichzeitig ist die Wiederverwendung verhindert.

### Bezugszeichenliste

- 01 -: Kanülenschutz
- 02 -: Hülse
- 03 -: Grundfläche
- 04 -: Öffnung
- 05 -: Kanüle
- 06 -: Faltenbalg
- 07 -: Vorsprung
- 08 -: Klemmfeder
- 09 -: Einwegspritze
- 10 -: radialer Überstand der Klemmfeder
- 11 -: Spritzenzylinder
- 12 -: Düse
- 13 -: Spritzenkolben
- 14 -: Betätigungsplatte
- 15 -: freies Ende des Faltenbalgs
- 16 -: Rasthaken
- 17 -: Rastnut
- 18 -: Sichtschlitz
- 19 -: Skala
- 20 -: Anschlagring des Spritzenzylinders
- 21 -: Hälften
- 22 -: Nase
- 23 -: Nut
- 24 -: -
- 25 -: -
- 26 -: Steg
- 27 -: Sollbruchkante

## Patentansprüche

1. Kanülenschutz (01) zur Abdeckung einer an einer Spritze (09) angebrachten Kanüle (05), umfassend:
- eine rohrförmige Hülse (02) zur axialbeweglichen Aufnahme der Spritze (09) mit einer Grundfläche (03) an ihrem einen Ende, die eine Öffnung (04) für den Durchlass der Kanüle aufweist;
- ein Federelement, das an dem anderen Ende der Hülse (02) angeformt ist, durch Einprägung einer Stauchungskraft gegen eine ihm immanente elastische Rückstellkraft axial um eine vorbestimmte Stauchungslänge stauchbar und nach Wegfall der Stauchungskraft selbsttätig rückstellend ist, mit einem freien Ende (15), zum Anschlag gegen einen Anschlagring (20) eines Spritzenzylinders (11) der Spritze (09), wenn dieser in die Hülse (02) verschoben wird, um die Kanüle (05) durch die Öffnung (04) hindurch nach außen zu schieben;
- mindestens eine radial wirkende Klemmfeder (08), welche an der Hülse (02) angeordnet ist;
**dadurch gekennzeichnet, dass** das Federelement ein Faltenbalg (06) ist, dass der kanülenschutz, umfassend Hülse, Faltenbalg und Klemmfeder einstückig oder in axialer Richdung zweigeteilt und über einen Rast mechanismus (22, 23) zusammensteckbar ausgebildet ist, und dass die Klemmfeder (08) infolge einer mittels Fingerdruck zweier die Hülse greifenden Finger radial eingeprägten Kraft gegen die in der Hülse (02) verschiebbare Spritze (09) drückbar ist, um diese bei gestauchtem Federelement festzuklemmen.

2. Kanülenschutz nach Anspruch 1, **dadurch gekennzeichnet, dass** die Hülsenlänge der Hülse (02) kürzer als die Länge des Spritzenzylinders (11) der Spritze (09) ist, die Stauchungslänge mindestens gleich der Länge der Kanüle (05) ist, die Summe aus Hülsenlänge und gestreckter Länge des Faltenbalgs (06) mindestens gleich der Summe aus Länge des Spritzenzylinders (11) und Länge der Kanüle (05) ist.

3. Kanülenschutz (01) nach Anspruch 1 oder 2, **dadurch gekennzeichnet, dass** der Faltenbalg (06) eine Schraubenform aufweist.

4. Kanülenschutz (01) nach einem der Ansprüche 1 bis 3, **dadurch gekennzeichnet, dass** vier Klemmfedern (08) jeweils paarweise radial gegenüberliegend an der Hülse (02) angeordnet sind.

5. Kanülenschutz (01) nach einem der Ansprüche 1 bis 4, **dadurch gekennzeichnet, dass** die Hülse (02) einen axial verlaufenden Schlitz (18) zur Sichtfreigabe auf die Spritze (09) aufweist, insbesondere auf eine Skala (19).

6. Kanülenschutz (01) nach einem der Ansprüche 1 bis 5, **dadurch gekennzeichnet, dass** er aus Kunststoff hergestellt ist.

7. Kanülenschutz (01) nach einem der Ansprüche 1 bis 6, **dadurch gekennzeichnet, dass** er durch Spritzgießen hergestellt ist.

8. Kanülenschutz (01) nach einem der Ansprüche 1 bis 7, **dadurch gekennzeichnet, dass** die Öffnung (04) eine kreisrunde Form oder die Form eines Kreuzes hat.

9. Kanülenschutz (01) nach einem der Ansprüche 1 bis 8, **dadurch gekennzeichnet, dass** er eine Rastnut (17) zur haltenden Aufnahme einer Einwegspritze (09) aufweist.

10. Einwegspritzensystem mit einem Kanülenschutz (01) nach einem der Ansprüche 1 bis 9 und mit einer Einwegspritze (09), welche einen Spritzenzylinder (11) mit einer Düse (12) zum Aufstecken einer Kanüle und einen Spritzenkolben (13) mit einer Betätigungsplatte (14) umfasst, wobei der Spritzenzylinder (11) über eine Verbindungsraste mit dem Kanülenschutz (01) arretierbar ist.

11. Einwegspritzensystem nach Anspruch 10, **dadurch gekennzeichnet, dass** der Spritzenkolben (13) mit einer Sollbruchkante (27) versehen ist, und dass im Inneren des Spritzenzylinders (11) nahe des Auslasses ein Steg (26) vorgesehen ist, welcher die Sollbruchkante (27) bei deren Auftreffen zerstört, so dass der Spritzenkolben (13) nachfolgend nicht mehr dichtend im Spritzenzylinder (11) gelagert ist.

12. Einwegspritzensystem nach Anspruch 10 oder 11, **dadurch gekennzeichnet, dass** an der Betätigungsplatte (14) des Spritzenkolbens (13) und an einem Anschlagring (20) des Spritzenzylinders (11) ein Verschlussmechanismus (16, 17) vorgesehen ist, der nach einmaligem vollständigen Einführen des Spritzenkolbens (13) in den Spritzenzylinder (11) ein Wiederherausziehen des Spritzenkolbens (13) verhindert.

13. Einwegspritzensystem nach einem der Ansprüche 10 bis 11, **dadurch gekennzeichnet, dass** an der Betätigungsplatte (14) des Spritzenkolbens (13) und am Kanülenschutz (01) ein Verschlussmechanismus (16, 17) vorgesehen ist, der nach einmaligem vollständigen Einführen des Spritzenkolbens (13) in den Spritzenzylinder (11) ein Entfernen der Spritze (09) aus dem Kanülenschutz (01) verhindert.

## Claims

1. Cannula protector (01) for covering a cannula (05) attached to a syringe (09), comprising:
- a tubular sleeve (02) for axially moveable accommodation of the syringe (09) with a base (03) at one of its ends, which has an opening (04) for the passage of the cannula;
- a spring element formed on the other end of the sleeve (02), which, by application of a compression force against an elastic resetting force that is intrinsic to it, can be compressed axially by a predetermined compression length, and which automatically resets when the compression force is removed, where the free end (15) of the spring element abuts against a stop ring (20) of a syringe cylinder (11) of the syringe (09), when it is moved into the sleeve (02), in order to move the cannula (05) outwards through the opening (04);
- at least one radial acting clamp spring (08) which is arranged on the sleeve (02);
**characterized in that** the spring element is a bellows (06), that the cannula protector comprising sleeve, bellows and clamp spring is designed in one-piece or axially two-piece divided and pluggable by an indexing mechanism (22, 23), and that the clamp spring (08) is, due to a radial applied force of two fingers gripping on the sleeve can be pushed against the syringe (09) which is moveable in the sleeve (02), in order to clamp it when the spring element is compressed.

2. Cannula protector (01) according to Claim 1, **characterized in that** the sleeve length of the sleeve (02) is shorter than the length of the syringe cylinder (11) of the syringe (09), the compression length is at least equal to the length of the cannula (05), the sum of the sleeve length and the stretched length of the bellows (06) is at least equal to the sum of the length of the syringe cylinder (11) and the length of the cannula (05).

3. Cannula protector (01) according to Claim 1 or 2, **characterized in that** the bellows (06) is in the shape of a screw.

4. Cannula protector (01) according to one of Claims 1-3, **characterized in that** four clamp springs (08) are arranged in each case in radially opposite pairs on the sleeve (02).

5. Cannula protector (01) according to one of Claims 1-4, **characterized in that** the sleeve (02) has an axially running slit (18) to expose the syringe (09), particularly a scale (19), to view.

6. Cannula protector (01) according to one of Claims 1-5, **characterized in that** it is manufactured from plastic.

7. Cannula protector (01) according to one of Claims 1-6, **characterized in that** it is manufactured by injection molding.

8. Cannula protector (01) according to one of Claims 1-7, **characterized in that** the opening (04) is in the shape of a circle or in the shape of a cross.

9. Cannula protector (01) according to one of Claims 1-8, **characterized in that** it presents an engagement groove (17) for retentive accommodation of a single-use syringe (09).

10. Single-use syringe system with a cannula protector (01) according to one of Claims 1-19 and with a single-use syringe (09) which comprises a syringe cylinder (11) with a nozzle (12) for the attachment of a cannula, and a syringe plunger (13) with an actuation plate (14), where the syringe cylinder (11) can be locked by means of a connecting notch with the cannula protector (01).

11. Single-use syringe system according to Claim 10, **characterized in that** the syringe plunger (13) is provided with a predetermined breaking edge (27), and, in the interior of the syringe cylinder (11), close to the outlet, a bar (26) is provided, which destroys the predetermined breaking edge (27) as it strikes, so that the syringe plunger (13) is subsequently no longer mounted with seal in the syringe cylinder (11).

12. Single-use syringe system according to Claim 10 or 11, **characterized in that**, on the actuation plate (14) of the syringe plunger (13), and on a stop ring (20) of the syringe cylinder (11), a closing mechanism (16, 17) is provided, which, after the one-time complete introduction of the syringe plunger (13) into the syringe cylinder (11), prevents the syringe plunger (13) from being pulled out again.

13. Single-use syringe system according to one of Claims 10-11, **characterized in that**, on the actuation plate (14) of the syringe plunger (13) and on the cannula protector (01), a closing mechanism (16, 17) is provided, which, after the one-time complete introduction of the syringe plunger (13) into the syringe cylinder (11), prevents the removal of the syringe (09) from the cannula protector (01).

## Revendications

1. Protection de canule (01) pour recouvrir une canule (05) placée sur une aiguille (09), comprenant :
- un manchon tubulaire (02) pour la réception en déplacement axial de l'aiguille (09) avec un fond (03) sur l'une de ses extrémités, qui présente une ouverture (04) pour le passage de la canule ;
- un élément de ressort formé sur l'autre extrémité du manchon (02), pouvant être refoulé axialement d'une longueur de refoulement prédéfinie, par l'exercice d'une force de refoulement contre une force de rappel élastique qui lui est immanente et avec un rappel automatique après la suppression de la force de refoulement, comprenant une extrémité libre (15), pour venir buter contre une bague de butée (20) d'un cylindre d'aiguille (11) de l'aiguille (09), lorsque celui-ci est poussé dans le manchon (02) pour pousser la canule (05) vers l'extérieur à travers l'ouverture (04) ;
- au moins un ressort de serrage (08) agissant radialement qui est placé sur le manchon (02) ;
**caractérisé en ce que** l'élément de ressort est un soufflet (06), que la protection de canule comprenant manchon, soufflet et ressort de serrage est monobloc ou divisée dans la direction axiale et peut être assemblée par un mécanisme d'encliquetage (22, 23), et que le ressort de serrage (08), à la suite d'une force exercée radialement au moyen d'une pression de deux doigts saisissant le manchon, peut être appuyé contre l'aiguille (09) pouvant être poussée dans le manchon (02) pour bloquer celle-ci lorsque l'élément de ressort est refoulé.

2. Protection de canule selon la revendication 1, **caractérisée en ce que** la longueur de manchon du manchon (02) est plus courte que la longueur du cylindre d'aiguille (11) de l'aiguille (09), la longueur de refoulement est au moins égale à la longueur de la canule (05), la somme de la longueur de manchon et de la longueur étirée du soufflet (06) est au moins égale à la somme de la longueur du cylindre d'aiguille (11) et de la longueur de la canule (05).

3. Protection de canule (01) selon la revendication 1 ou 2, **caractérisée en ce que** le soufflet (06) présente une forme hélicoïdale.

4. Protection de canule (01) selon l'une des revendications 1 à 3, **caractérisée en ce que** quatre ressorts de serrage (08) radialement opposés par paire respective sont disposés sur le manchon (02).

5. Protection de canule (01) selon l'une des revendications 1 à 4, **caractérisée en ce que** le manchon (02) présente une fente (18) au tracé axial pour exposer l'aiguille (09), en particulier une échelle (19).

6. Protection de canule (01) selon l'une des revendications 1 à 5, **caractérisée en ce qu'**elle est fabriquée en plastique.

7. Protection de canule (01) selon l'une des revendications 1 à 6, **caractérisée en ce que** qu'elle est fabriquée par moulage par injection.

8. Protection de canule (01) selon l'une des revendications 1 à 7, **caractérisée en ce que** l'ouverture (04) présente une forme circulaire ou la forme d'une croix.

9. Protection de canule (01) selon l'une des revendications 1 à 8, **caractérisée en ce qu'**elle présente une rainure d'encliquetage (17) pour recevoir et retenir une aiguille à usage unique (09).

10. Système d'aiguille à usage unique comprenant une protection de canule (01) selon l'une des revendications 1 à 9 et comprenant une aiguille à usage unique (09) qui comprend un cylindre d'aiguille (11) avec un injecteur (12) pour enficher une canule et un piston d'aiguille (13) avec un plateau d'actionnement (14), sachant que le cylindre d'aiguille (11) peut être bloqué avec la protection de canule (01) par le biais d'une encoche de liaison.

11. Système d'aiguille à usage unique selon la revendication 10, **caractérisé en ce que** le piston d'aiguille (13) est muni d'une arête de rupture (27) et **en ce qu'**à l'intérieur du cylindre d'aiguille (11) près de la sortie, une branche (26) est prévue qui détruit l'arête de rupture (27) lorsqu'elle se présente, de façon à ce que le piston d'aiguille (13) ne soit ensuite plus placé de manière hermétique dans le cylindre d'aiguille (11).

12. Système d'aiguille à usage unique selon la revendication 10 ou 11, **caractérisé en ce qu'**un mécanisme de fermeture (16, 17) est prévu sur le plateau d'actionnement (14) du piston d'aiguille (13) et sur une bague de butée (20) du cylindre d'aiguille (11), lequel, après l'introduction complète unique du piston d'aiguille (13) dans le cylindre d'aiguille (11), empêche de ressortir le piston d'aiguille (13).

13. Système d'aiguille à usage unique selon l'une des revendications 10 à 11, **caractérisé en ce qu'**un mécanisme de fermeture (16, 17) est prévu sur le plateau d'actionnement (14) du piston d'aiguille (13) et sur la protection de canule (01), lequel, après l'introduction complète unique du piston d'aiguille (13) dans le cylindre d'aiguille (11), empêche de retirer l'aiguille (09) de la protection de canule (01).
